# FASCICULE DE BREVET EUROPEEN

(11) **EP 3 297 626 B1**
(45) Date de publication et mention de la délivrance du brevet: **16.06.2021**
(21) Numéro de dépôt: 16727328.3
(22) Date de dépôt: 20.05.2016
(51) Int. Cl.: A61K 31/385, A61K 9/00, A61K 31/715, A61K 31/728, A61P 27/02, A61K 45/06

(54) **COMPOSITION OPHTALMIQUE COMPRENANT DE L'ACIDE LIPOIQUE ET DE L'ACIDE HYALURONIQUE**
OPHTHALMISCHE ZUSAMMENSETZUNGEN MIT LIPONSÄURE UND HYALURONSÄURE
OPHTHALMIC COMPOSITION COMPRISING LIPOIC ACID AND HYALURONIC ACID

(30) Priorité: 21.05.2015 FR 1554590; 11.03.2016 FR 1652042
(43) Date de publication de la demande: 28.03.2018
(73) Titulaire: Ophtalmis Monaco, 98000 Monaco (MC)
(72) Inventeur: CLARET, Martine, 1025 Saint Sulpice (CH); CLARET, Claude, 1025 Saint Sulpice (CH); CHATARD-BAPTISTE, Caroline, 06100 Nice (FR)
(74) Mandataire: Be IP
(86) Numéro de dépôt international: PCT/EP2016/061362
(87) Numéro de publication internationale: WO 2016/185000

(56) Documents cités:
- WO-A1-2006/128618
- WO-A1-2009/080220
- WO-A2-2011/131765
- US-A1- 2006 216 251
- US-A1- 2007 207 116
- KOFUJI ET AL: "Stabilization of alpha-lipoic acid by complex formation with chitosan", FOOD CHEMISTRY, ELSEVIER LTD, NL, vol. 109, no. 1, 14 décembre 2007 (2007-12-14), pages 167-171, XP022480119, ISSN: 0308-8146, DOI: 10.1016/J.FOODCHEM.2007.11.078

## Description

### DOMAINE DE L'INVENTION

La présente invention concerne une composition ophtalmique sous forme d'une émulsion huile dans eau comprenant un polymère muco-mimétique choisi parmi l'acide hyaluronique et ses sels et de l'acide lipoïque comme stabilisant de l'émulsion. Elle concerne aussi un procédé de stabilisation des émulsions avec de l'acide lipoïque et l'utilisation de cet acide pour stabiliser les émulsions huile dans eau comprenant un polymère muco-mimétique choisi parmi l'acide hyaluronique et ses sels.

### ETAT DE LA TECHNIQUE

On connaît des émulsions comprenant des polymères mucomimétiques, notamment des compositions ophtalmiques sous forme de telles émulsions (WO 2011/131765, WO 2012/013736, WO 2015/150459). Toutefois, de telles émulsions, en particulier en absence de tout agent conservateur, peuvent présenter des problèmes de stabilité dans le temps, réduisant l'homogénéité de la composition et son efficacité à l'usage.

L'acide lipoïque, également appelé acide thioctique (CAS n° 0001077-28-7) et ses dérivés sont connus pour leurs propriétés antioxydantes. Il est employé comme complément alimentaire sous forme de comprimés (Liponsäure-ratiopharm® commercialisé en Allemagne par la société Ratiopharm) ou encore en injection sous forme de solutions diluées injectables (Neurium® commercialisé en Allemagne par la société Hexal), éventuellement dans l'accompagnement de traitements de patients diabétiques. Ses propriétés antimicrobiennes ont été également décrites pour son utilisation dans des solutions de nettoyage de lentilles à base de BDT (US 6 162 393). Les propriétés antioxydantes de l'acide lipoïque ont été également mises en avant pour étudier son efficacité sur la cataracte diabétique chez le rat (Masami Kojima & col., Japanese Journal of Ophthalmology, January 2007, Volume 51, Issue 1, pp 10-13). Des compositions ophtalmiques ou cosmétiques pouvant comprendre de l'acide lipoïque comme agent antioxydant sont décrites dans des demandes de brevet (US 2006/188492, US 2004/265345, US 5 817 630, WO 02098345, DE10229995, WO 01/93824, US 2005/192229, BR PI0 800 818, CN 103 860 625 et JP 2013 241398).

Les inventeurs ont maintenant constaté que l'ajout d'acide lipoïque à une émulsion huile dans eau comprenant un polymère muco-mimétique permettait d'améliorer la stabilité de l'émulsion.

### EXPOSE DE L'INVENTION

La présente invention concerne une composition ophtalmique sous forme d'une émulsion huile dans eau comprenant un polymère mucomimétique choisi parmi l'acide hyaluronique et ses sels et de l'acide lipoïque. L'acide lipoïque a pour effet de stabiliser l'émulsion.

L'invention concerne plus particulièrement une composition ophtalmique sous forme d'une émulsion huile dans eau comprenant de l'acide hyaluronique ou ses sels comme polymère mucomimétique et de l'acide lipoïque.

Sauf indication contraire, les pourcentages sont exprimés en poids par rapport au poids total de la composition.

L'invention concerne également un procédé pour stabiliser une émulsion huile dans eau comprenant de l'acide hyaluronique ou ses sels, comprenant l'addition d'acide lipoïque avant ou pendant l'étape d'émulsification de la composition.

L'invention concerne aussi l'utilisation de l'acide lipoïque pour stabiliser une émulsion huile dans eau comprenant de l'acide hyaluronique ou ses sels.

### DESCRIPTION DETAILLEE DE L'INVENTION

La présente invention concerne une composition ophtalmique sous forme d'une émulsion huile dans eau comprenant un polymère muco-mimétique choisi parmi l'acide hyaluronique et ses sels et de l'acide lipoïque comme stabilisant de l'émulsion.

Par composition ophtalmique, on entend selon l'invention tout composition destinée à être appliquée sur l'œil ou la muqueuse oculaire. Les compositions ophtalmiques peuvent être des compositions visqueuses telles que des pommades ou crèmes, ou bien des compositions liquides, dites collyres.

La composition ophtalmique doit répondre à des caractéristiques techniques spécifiques des compositions ophtalmiques, et plus particulièrement liées à la sélection de ses composants. Ces composants qui sont « ophtalmiquement acceptables » ne doivent pas, individuellement ou associés dans la composition, provoquer de réactions secondaires de l'œil en dehors de l'effet recherché par la composition et ses actifs. L'œil étant un organe particulièrement sensible à un stress environnemental, la composition ne doit pas provoquer d'irritations parasites ou de réactions de type allergiques au détriment recherché, plus particulièrement dans le cas de compositions ophtalmiques destinées à traiter une affection ophtalmique. Le choix des constituants de la composition est donc très important, qui distingue la composition ophtalmique d'une simple composition inadaptée pour un usage ophtalmique. L'homme du métier est à même de choisir lesdits composants et de différencier une composition ophtalmique d'une simple composition destinée à un autre usage.

Les polymères muco-mimétiques sont des polyosides, en particulier choisi parmi les polysaccharides, encore appelés glycanes ou polyholosides, et plus particulièrement parmi les glycosaminoglycanes (GAG) et les acides hyaluroniques. Ces polymères, également désignés sous le terme mucopolysaccharides acides, peuvent être caractérisés par une forte capacité de rétention de l'eau leur conférant des propriétés muco mimétiques. Le polymère mucomimétique est choisi parmi les acides hyaluroniques et ses sels.

Le polymère muco-mimétique peut avoir un poids moléculaire allant de 10 à 10 000 kDa, notamment de 500 à 1 500 kDa, voire d'environ 1 000 kDa.

L'émulsion selon l'invention comprend une teneur en polymère mucomimétique allant de 0,01 à 5 % en poids par rapport au poids total de la composition, notamment allant de 0,05 à 2,5 % en poids, en particulier allant de 0,1 à 1 % en poids, voire allant de 0,15 à 0,5 % en poids, et encore plus particulièrement d'environ 0,18 % en poids par rapport au poids total de l'émulsion

. Les différentes formes d'acide hyaluronique et ses sels employés dans des compositions pharmaceutiques ou cosmétiques sont bien connues de l'homme du métier et sont susceptibles d'être employées dans les compositions selon l'invention.

De préférence, l'acide hyaluronique est employé sous sa forme de sel de métal alcalin, en particulier son sel de sodium. On citera notamment le hyaluronate de sodium avec une viscosité intrinsèque allant de 1,4 à 2,2 m3/kg.

De préférence l'émulsion selon l'invention a une teneur en acide hyaluronique ou ses sels allant de 0,05 à 2 % en poids par rapport au poids total de la composition, en particulier allant de 0,1 à 0,2 % en poids.

Par acide lipoïque, on entend selon l'invention l'acide 5-(1,2-dithiolan-3-yl)pentanoïque, sous forme racémique ou ses énantiomères en toutes proportions, en particulier l'énantiomère R, pur ou en mélange où la proportion d'énantiomère R est supérieure à celle de l'énantiomère S, et leurs sels pharmaceutiquement acceptables.

Parmi les sels pharmaceutiquement acceptables, on entend selon l'invention avantageusement les sels d'addition de l'acide lipoïque avec une base pharmaceutiquement acceptable, qu'il s'agisse d'une base organique notamment comprenant un groupe amino, comme l'ammoniac, la lysine, l'arginine et autres composés connus de la pharmacopée ou avec une base inorganique pharmaceutiquement acceptable telle que la soude, la potasse, l'hydroxyde de calcium et d'autres bases inorganiques connues de la pharmacopée. Préférentiellement, le sel pharmaceutiquement acceptable est un sel de métal alcalin (sodium, potassium), de métal alcalino-terreux (calcium, magnésium) ou un ion d'aluminium, plus préférentiellement un sel de sodium.

Selon un mode préférentiel de réalisation de l'invention, l'acide lipoïque est un sel de l'énantiomère R de l'acide lipoïque, en particulier sel de sodium (CAS 176110-81-9) ou de magnésium.

De manière avantageuse, la teneur en acide lipoïque dans l'émulsion selon l'invention va de 0,01 à 0,02 % en poids.

De préférence, le rapport pondéral acide lipoïque / acide hyaluronique va de 0,05 à 0,15.

La composition est une émulsion huile dans eau et comprend les constituants usuels de telles émulsions, comme les corps gras, les solvants organiques, les tensioactifs ioniques, zwitterioniques ou non-ioniques et les agents émulsifiants. Les constituants des émulsions en particulier employées pour une application sur la peau ou les muqueuses sont bien connus de l'homme du métier. On citera notamment :
- des agents stabilisants de l'émulsion, en particulier des polymères stabilisants, tels que les polymères d'alcool de polyvinyle, polysorbates, et de type cellulose, notamment la méthylcellulose, l'éthylcellulose, l'hydroxypropylcellulose, la carboxyméthylcellulose, et leurs mélanges ; de tels agents stabilisants peuvent être employés à des teneurs allant de 0,05 et 0,2% en poids, par rapport au poids total de la composition ;
- des agents tensioactifs, notamment les polysorbates, les polyéthylènes glycol et leurs dérivés, le polyoxyéthylène-40-stéarate, les esters de sorbitane, les copolymères polyoxyéthylène-polyoxypropylène, les alcools polyvinyliques, les polymères de polyvinylpirrolidone, et leurs mélanges ; de tels agents tensioactifs peuvent être employés à des teneurs allant de 0,01 et 0,5% en poids, par rapport au poids total de la composition ;
- des agents conservateurs, en particulier, les ammoniums quaternaires, notamment chlorure de benzalkonium, alkyldiméthylbenzylammonium, cétrimide, chlorure de cétylpyridinium, bromure de benzododécinium, chlorure de benzothonium, chlorure de cetalkonium, les conservateurs mercuriels, comme nitrate/acétate/borate phénylmercurique, thiomersal, les conservateurs alcooliques, comme chlorobutanol, alcool benzylique, phényléthanol, alcool phénylethylique, les acides carboxyliques, tels qu'acide sorbique, les phénols, en particulier méthyl/propyl parabène, les amidines, par exemple chlorhexidine digluconate, l'EDTA, agent chélateur potentialisant l'efficacité des conservateurs, en association avec au moins un conservateur, et leurs mélanges ; de tels agents conservateurs peuvent être employés à des teneurs allant de 0,001 et 0,1% en poids, par rapport au poids total de la composition.
- des lipides, notamment des triglycérides et leurs dérivés, comme les lipides de la classe des acylglycérol ou glycéride, tel que les huiles de ricin, de soja, de sésame, de paraffine, de lanoline, de vaseline, de mais, de glycérine, ou de monoglycéride, de triglycérides, de type phospholipides, comme les phosphoacylglycérides, encore appelés phosphacylglycérols, les phosphosphingolipides, les phosphonoshingolipides les phosphoglycolipides, phosphosacharolipides et les phosphatidylcholines, et leurs mélanges ; ces lipides peuvent être employés à des teneurs allant de 0,1 et 1,5 % en poids, par rapport au poids total de la composition ;
- un tampon pour stabiliser le pH à une valeur acceptable pour son utilisation sur l'oeil, en particulier un tampon phosphate, acétate, citrate, et leurs mélanges ; ce tampon peut être employé à une teneur allant de 0,1 et 1,5 % en poids, par rapport au poids total de la composition.

Les compositions de l'invention peuvent comprendre d'autres adjuvants usuels mis en œuvre pour la préparation de telles formulations, les adoucissants, les antioxydants, les opacifiants, les stabilisants, les épaississants ioniques ou non ioniques, les silicones, les agents anti-mousse, les agents hydratants, les vitamines, les parfums, les conservateurs, les charges, les séquestrants, les colorants, les bases ou les acides nécessaires à la régulation du pH, ou tout autre ingrédient habituellement utilisé pour la préparation de compositions ophtalmiques. Les compositions ophtalmiques selon l'invention sont préparées selon les techniques bien connues de l'homme du métier.

Selon un mode préféré de réalisation de l'invention, la composition ophtalmique selon l'invention est une composition sans conservateurs.

Les agents conservateurs généralement employés dans des compositions topiques (cosmétiques, pharmaceutiques, ophtalmiques, etc.) pour éviter leur contamination par des germes, sont bien connus de l'homme du métier, comme les ammoniums quaternaires, notamment le chlorure de benzalkonium, l'alkyl-diméthyl-benzylammonium, le cétrimide, le chlorure de cétylpyridinium, le bromure de benzododécinium, le chlorure de benzothonium, le chlorure de cetalkonium, les conservateurs mercuriels, comme le nitrate/acétate/borate phénylmercurique, le thiomersal, les conservateurs alcooliques, comme le chlorobutanol, l'alcool benzylique, le phényléthanol, l'alcool phénylethylique, les acides carboxyliques, tels que l'acide sorbique, les phénols, en particulier méthyl/propyl parabène, les amidines, par exemple le chlorhexidine digluconate et/ou des agents chélateur comme l'EDTA seul ou en association avec au moins un autre conservateur.

Par « sans conservateurs », on entend selon l'invention une composition substantiellement dépourvue de tels conservateurs pour répondre à une indication « sans conservateurs ». Sa teneur en conservateurs est inférieure ou égale à 10 ppm, plus particulièrement inférieure ou égale à 1 ppm, préférentiellement égale à 0 ppm, aucun conservateur n'entrant dans sa composition.

En l'absence de conservateurs, la composition doit subir un traitement particulier au cours de sa préparation et de son conditionnement de manière à éviter et prévenir la contamination par des pathogènes. Ces traitements et procédures sont bien connus de l'homme du métier. En ce sens, une composition sans conservateurs selon l'invention se distingue d'une simple composition comprenant les mêmes ingrédients obtenue sans montrer de précautions particulières ni décrivant d'étapes du procédé permettant d'obtenir cette stérilité caractéristique des compositions selon l'invention, en particulier des compositions ophtalmiques.

La composition ophtalmique a de préférence un pH compris entre 6 et 7. Elle comprend donc généralement un tampon approprié pour un usage ophtalmique, connu de l'homme du métier. On citera en particulier le citrate trisodique dihydraté et l'acide citrique monohydraté employés seuls ou en mélange.

La composition ophtalmique doit également être stérile pour ne pas apporter de pathogènes susceptibles de se développer et entrainer des complications ophtalmiques. Par « stérile », on entend au sens de la présente invention l'absence de germes au sens de la Pharmacopée Européenne, 8ème édition (2014). De manière préférentielle, la composition ophtalmique qui comprend de l'acide lipoïque selon l'invention est une composition sans conservateurs.

Selon un mode de réalisation de l'invention, la composition ophtalmique comprend des composés dits actifs, comme un agent osmoprotecteur, un agent anti-inflammatoire et/ou un agent anti-oxydant.

Parmi les agents osmoprotecteurs susceptibles d'être employés selon l'invention, on citera la glycérine, la taurine, la L-Carnitine, l'érythritol, le tréhalose, l'ectoïne, la bétaïne, la sarcosine, l'urée, de préférence la glycérine ou la taurine.

Parmi les agents à activité anti-inflammatoire susceptibles d'être employés avec l'acide lipoïque, on citera la dexaméthasone, le flurbiprofène, la fluorométholone, l'acide salicylique, l'hydrocortisone, la triamcinolone, la rimexolonede préférence le flurbiprofène et la dexaméthasone.

Parmi les agents antioxydants susceptibles d'être employés on citera la taurine, la vitamine E, le glutathion, la vitamine A, la vitamine C, de préférence la taurine.

La viscosité de l'émulsion est avantageusement choisie de manière à permettre son maintien sur l'œil, en particulier sur la cornée pendant un temps suffisant pour lui permettre d'agir.

La composition ophtalmique selon l'invention a de préférence une viscosité allant de 5 à 100 centipoises. Cette viscosité est mesurée selon les préconisations de la Pharmacopée Européenne 2.2.10, avec un viscosimètre rotatif, à 25°C, et 100s-1. D'autres appareils de mesure et méthodes adaptés à la mesure de la viscosité de solutions sont connus de l'homme du métier et permettent d'obtenir des résultats similaires.

De manière avantageuse, l'émulsion selon l'invention présente une faible viscosité proche de celle de l'eau, notamment une viscosité dynamique inférieure ou égale à 10"1 Pa.s, en particulier allant de 1 ,5.10-3 à 8.10"2, et tout particulièrement allant de 3.10"3 à 6.10-2 Pa.s.

La viscosité de la composition ophtalmique selon l'invention est adaptée par l'ajout d'agents de viscosité « ophtalmiquement acceptables ». L'homme du métier connait bien les agents de viscosité susceptibles d'être employés pour la préparation de compositions ophtalmiques et les quantités à employer pour obtenir la viscosité recherchée. On citera en particulier l'hydroxypropylméthylcellulose, l'hydroxyethylcellulose, la carboxymethylcellulose, carbomères, les gels agar, polyvinylpyrrolidone et l'alcool polyvinylique.

De manière préférentielle, la composition ophtalmique selon l'invention comprend un polymère stabilisant de type cellulose, notamment la méthylcellulose, l'hydroxypropylméthylcellulose, préférentiellement à une teneur allant de 0,05 à 0,5 % en poids, avantageusement de 0,1 à 0,4 % en poids, plus avantageusement de 0,1 à 0,3 % en poids, particulièrement environ 0,15 % en poids.

L'émulsion selon l'invention présente avantageusement une limpidité telle que définie par European Pharmacopeia 7.0 allant de 0 à 5000 NTU, en particulier allant de 0 à 1000 NTU, tout particulièrement d'environ 200 à 700 NTU, voire d'environ 500 NTU.

De manière préférée, l'émulsion selon l'invention est un collyre. En particulier, il comprend au moins 70 % d'eau en poids par rapport au poids total de la composition, préférentiellement au moins 80 % d'eau, plus préférentiellement au moins 95 % d'eau.

Les compositions ophtalmiques selon l'invention sont préparées selon les méthodes usuelles connues de l'homme du métier. Ces méthodes comprennent une étape d'émulsification après mélange des constituants de la composition. On peut parfois préparer les deux phases aqueuse et lipidique séparément, notamment lorsqu'il faut dissoudre un ingrédient particulier dans l'une des phases avant de les mélanger. L'invention concerne également un procédé de préparation d'une émulsion huile dans eau comprenant de l'acide hyaluronique ou ses sels et de l'acide lipoïque, avec l'addition d'acide lipoïque avant ou pendant l'étape d'émulsification de la composition comprenant de l'acide hyaluronique ou ses sels.

L'acide lipoïque est avantageusement ajouté à une phase lipidique.

L'invention concerne aussi l'utilisation de l'acide lipoïque pour stabiliser une émulsion huile dans eau comprenant de l'acide hyaluronique ou ses sels.

### EXEMPLES

### 1. Compositions

Les compositions données en exemples ci-après sont préparées selon les méthodes usuelles du domaine technique. Des précautions sanitaires particulières sont prises pour la préparation et le conditionnement des compositions sans conservateurs de manière à éviter les contaminations.

**Émulsion 1**

| **Composants** | **Quantité** |
|---|---|
| Hyaluronate de sodium | 0.18 % |
| Lipoic acid | 0.002% |
| Caprylic/Capric Triglyceride | 0,10% |
| Lécithine de soja | 0,10% |
| Carboxyméthylcellulose Sodique | 0,10% |
| Citrate trisodique dihydraté | 0.41% |
| Acide citrique | 0.02% |
| NaOH (1 N) | Qsp pH 6.7 |
| Chlorure de sodium | Qsp 150mOsmol/L |
| Eau ppi | Qsp 100 % |

**Émulsion 2**

| **Composants** | **Quantité** |
|---|---|
| Hyaluronate de sodium | 0.18% |
| Lipoic acid | 0.002% |
| Caprylic/Capric Triglyceride | 0,165% |
| Lécithine de soja | 0,035% |
| Hydroxyméthylcellulose | 0,10% |
| Citrate trisodique dihydraté | 0.41% |
| Acide citrique | 0.02% |
| NaOH (1 N) | Qsp pH 6.7 |
| Chlorure de sodium | Qsp 150mOsmol/L |
| Eau ppi | Qsp 100 % |

### 2. Stabilité

Des premiers lots testés en laboratoire ont permis de mettre en évidence une amélioration de la stabilité des émulsions contenant de l'acide lipoïque. La stabilité des émulsions est notamment représentée par la taille des particules. La capacité d'une émulsion à maintenir des particules de petite taille est un marqueur de stabilité. Des mesures de taille de particules ont été faites sur des formules avec et sans acide lipoïque, les autres ingrédients étant par ailleurs identiques.

Les particules restent plus petites pour des émulsions avec de l'acide lipoïque que sans, ce qui confirme l'amélioration de la stabilité par l'apport d'acide lipoïque.

Des études complémentaires sont planifiées sur 24 mois (test à 1/3/6/12 mois, 18 mois et 24 mois) à 25, 30 et 40°C.

Sont observés et mesurés : l'apparence, la taille des particules, la viscosité, le pH, l'osmolalité et le potentiel zeta.

La plus grande stabilité des émulsions comprenant de l'acide lipoïque selon l'invention est attendue avec une diminution de la taille des particules, un potentiel zêta inférieur à 50 et une plus grande stabilité dans le temps.

### REFERENCES

- BR PI0 800 818
- CN 103 860 625
- DE10 229 995
- JP 2013 241398
- US 5 817 630
- US 6 162 393
- US 2004/265345
- US 2005/192229
- US 2006/188492
- WO 01/93824
- WO 02/098345
- WO 2011/131765
- WO 2012/013736
- WO 2015/150459
- Masami Kojima & col., Japanese Journal of Ophthalmology, January 2007, Volume 51, Issue 1, pp 10-13

## Revendications

1. Composition ophtalmique sous forme d'une émulsion huile dans eau comprenant un polymère muco-mimétique choisi parmi l'acide hyaluronique et ses sels et de l'acide lipoïque.

2. Composition selon la revendication 1, **caractérisée en ce que** le polymère muco-mimétique est un sel d'acide hyaluronique.

3. Composition selon l'une des revendications 1 ou 2, **caractérisée en ce que** la teneur en acide hyaluronique ou ses sels va de 0,01 à 5 % en poids par rapport au poids total de la composition.

4. Composition selon l'une des revendications 1 à 3, **caractérisée en ce que** l'acide lipoïque est le lipoate de sodium.

5. Composition selon l'une des revendications 1 à 4, **caractérisée en ce que** la teneur en polymère acide lipoïque va de 0,01 à 0,02 % en poids par rapport au poids total de la composition.

6. Composition selon l'une des revendications 1 à 5, **caractérisée en ce que** le rapport pondéral acide lipoïque/acide hyaluronique ou ses sels va de 0,05 à 0,15.

7. Composition selon l'une des revendications 1 à 6, **caractérisée en ce qu'**elle a une limpidité inférieure ou égale à 5000 NTU.

8. Composition selon la revendication 7, **caractérisée en ce qu'**elle a une limpidité inférieure ou égale à 1000 NTU.

9. Composition selon la revendication 8, **caractérisée en ce qu'**elle a une limpidité d'environ 200 à 700 NTU.

10. Composition selon l'une des revendications 1 à 9, **caractérisée en ce qu'**elle est sous forme d'un collyre.

11. Composition selon la revendication 10, **caractérisée en ce qu'**elle comprend au moins 70 % d'eau en poids par rapport au poids total de la composition.

12. Composition selon la revendication 11, **caractérisée en ce qu'**elle comprend au moins 80 % d'eau, plus préférentiellement au moins 95 % d'eau en poids par rapport au poids total de la composition.

13. Procédé de préparation d'une émulsion huile dans eau comprenant de l'acide hyaluronique ou ses sels et de l'acide lipoïque selon l'une des revendications 1 à 12, **caractérisé en ce qu'**il comprend l'addition d'acide lipoïque avant ou pendant l'étape d'émulsification de la composition comprenant de l'acide hyaluronique ou ses sels.

14. Utilisation de l'acide lipoïque pour stabiliser une émulsion huile dans eau comprenant de l'acide hyaluronique ou ses sels.

15. Utilisation selon la revendication 14, **caractérisée en ce que** l'émulsion est définie selon l'une des revendications 2 à 12.

## Patentansprüche

1. Ophthalmische Zusammensetzung in der Form einer Öl-in-Wasser-Emulsion, umfassend ein mukomimetisches Polymer, das aus Hyaluronsäure und ihren Salzen und Liponsäure ausgewählt ist.

2. Zusammensetzung nach Anspruch 1, **dadurch gekennzeichnet, dass** das mukomimetische Polymer ein Salz von Hyaluronsäure ist.

3. Zusammensetzung nach einem der Ansprüche 1 oder 2, **dadurch gekennzeichnet, dass** der Gehalt an Hyaluronsäure oder ihren Salzen von 0,01 bis 5 Gew.-%, bezogen auf das Gesamtgewicht der Zusammensetzung, reicht.

4. Zusammensetzung nach einem der Ansprüche 1 bis 3, **dadurch gekennzeichnet, dass** die Liponsäure Natriumlipoat ist.

5. Zusammensetzung nach einem der Ansprüche 1 bis 4, **dadurch gekennzeichnet, dass** der Gehalt an Liponsäure-Polymer von 0,01 bis 0,02 Gew.-%, bezogen auf das Gesamtgewicht der Zusammensetzung, reicht.

6. Zusammensetzung nach einem der Ansprüche 1 bis 5, **dadurch gekennzeichnet, dass** das Gewichtsverhältnis von Liponsäure/Hyaluronsäure oder ihren Salzen von 0,05 bis 0,15 reicht.

7. Zusammensetzung nach einem der Ansprüche 1 bis 6, **dadurch gekennzeichnet, dass** sie eine Klarheit aufweist, die kleiner als oder gleich 5000 NTU ist.

8. Zusammensetzung nach Anspruch 7, **dadurch gekennzeichnet, dass** sie eine Klarheit aufweist, die kleiner als oder gleich 1000 NTU ist.

9. Zusammensetzung nach Anspruch 8, **dadurch gekennzeichnet, dass** sie eine Klarheit von ungefähr 200 bis 700 NTU aufweist.

10. Zusammensetzung nach einem der Ansprüche 1 bis 9, **dadurch gekennzeichnet, dass** sie in der Form von Augentropfen ist.

11. Zusammensetzung nach Anspruch 10, **dadurch gekennzeichnet, dass** sie mindestens 70 Gew.-% Wasser, bezogen auf das Gesamtgewicht der Zusammensetzung, umfasst.

12. Zusammensetzung nach Anspruch 11, **dadurch gekennzeichnet, dass** sie mindestens 80 Gew.-% Wasser, mehr bevorzugt mindestens 95 Gew.-% Wasser, bezogen auf das Gesamtgewicht der Zusammensetzung, umfasst.

13. Verfahren zur Herstellung einer Öl-in-Wasser-Emulsion von Hyaluronsäure oder ihren Salzen und Liponsäure nach einem der Ansprüche 1 bis 12, **dadurch gekennzeichnet, dass** es die Zugabe von Liponsäure vor oder während dem Schritt einer Emulgierung der Zusammensetzung, umfassend Hyaluronsäure oder ihre Salze, umfasst.

14. Verwendung der Liponsäure zum Stabilisieren einer Öl-in-Wasser-Emulsion, umfassend Hyaluronsäure oder ihre Salze.

15. Verwendung nach Anspruch 14, **dadurch gekennzeichnet, dass** die Emulsion nach einem der Ansprüche 2 bis 12 definiert ist.

## Claims

1. An ophthalmic composition in the form of an oil-in-water emulsion comprising a mucomimetic polymer selected from hyaluronic acid and salts thereof and lipoic acid.

2. The composition according to claim 1, **characterized in that** the mucomimetic polymer is a salt of hyaluronic acid.

3. The composition according to claim 1 or 2, **characterized in that** the content of hyaluronic acid or salts thereof ranges from 0.01 to 5% by weight based on the total weight of the composition.

4. The composition according to any one of claims 1 to 3, **characterized in that** the lipoic acid is sodium lipoate.

5. The composition according to any one of claims 1 to 4, **characterized in that** the content of lipoic acid polymer ranges from 0.01 to 0.02% by weight based on the total weight of the composition.

6. The composition according to any one of claims 1 to 5, **characterized in that** the weight ratio of lipoic acid to hyaluronic acid or salts thereof ranges from 0.05 to 0.15.

7. The composition according to any one of claims 1 to 6, **characterized in that** it has a clarity of less than or equal to 5000 NTU.

8. The composition according to claim 7, **characterized in that** it has a clarity of less than or equal to 1000 NTU.

9. The composition according to claim 8, wherein it has a clarity of about 200 to 700 NTU.

10. The composition according to any one of claims 1 to 9, **characterized in that** it is in the form of eye drops.

11. The composition according to claim 10, **characterized in that** it comprises at least 70% of water by weight relative to the total weight of the composition.

12. The composition according to claim 11, **characterized in that** it comprises at least 80% water, more preferentially at least 95% water by weight relative to the total weight of the composition.

13. A process for preparing an oil-in-water emulsion comprising hyaluronic acid or salts thereof and lipoic acid according to any one of claims 1 to 12, **characterized in that** it comprises adding lipoic acid before or during the step of emulsifying the composition comprising hyaluronic acid or salts thereof.

14. Use of lipoic acid to stabilize an oil-in-water emulsion comprising hyaluronic acid or salts thereof.

15. Use according to claim 14, **characterized in that** the emulsion is defined according to one of claims 2 to 12.
